# NEUE EUROPÄISCHE PATENTSCHRIFT

(11) **EP 0 156 199 B2**
(45) Veröffentlichungstag und Bekanntmachung des Hinweises auf die Entscheidung über den Einspruch: **23.11.1994**
(45) Hinweis auf die Patenterteilung: 26.10.1988
(21) Anmeldenummer: 85102445.5
(22) Anmeldetag: 05.03.1985
(51) Int. Cl.: C07C 205/06, C07C 201/06

(54) **Verfahren zur Herstellung von Nitrobenzol**
Process for the preparation of nitrobenzene
Procédé pour la préparation de nitrobenzène

(30) Priorität: 16.03.1984 DE 3409717
(43) Veröffentlichungstag der Anmeldung: 02.10.1985
(73) Patentinhaber: BAYER AG, 51368 Leverkusen (DE)
(72) Erfinder: Lailach, Günther, Dr., D-4150 Krefeld (DE); Gerken, Rudolf, Dr., D-4150 Krefeld (DE); Schultz, Karl-Heinz, Dr., D-4150 Krefeld (DE); Hornung, Rudolf, Dipl.-Ing., D-4150 Krefeld (DE); Böckmann, Walter, Dr., D-4150 Krefeld 11 (DE); Larbig, Wolfgang, Dr., D-4150 Krefeld (DE); Dietz, Wolfgang, Dipl.-Ing., D-4150 Krefeld 1 (DE)

(56) Entgegenhaltungen:
- EP-A- 0 016 987
- US-A- 2 256 999
- US-A- 4 091 042
- US-A- 4 091 042
- US-A- 4 241 229
- ULLMANS ENCYCLOPÄDIE DER TECHNISCHEN CHEMIE, 1982, 4. Auflage, 21, Seiten 150, 151 und 156
- WINNACKER/KÜCHLER CHEMISCHE TECHNOLOGIE, 1982, 2, Seiten 70-72
- ULLMANS ENCYCLOPÄDIE DER TECHNISCHEN CHEMIE, 1982, 4. Auflage, 17, Seiten 386, 387 und 391
- URBANSKI, CHEMIE UND TECHNOLOGIE DER EXPLOSIVSTOFFE 1961, 1, Seite 92
- Verbesserte Aromaten-Nitrierung, Chem.-Ing.-Techn., 1979, 51, Nr. 5
- Prospekt BOFORS NOBEL CHEMATUR, 1979
- Martin, Swarbrik, Cammarata, PHYSIKALISCHE PHARMAZIE, 2. Auflage, Seiten 103 und 104
- RÖMPPS CHEMIE LEXIKON, 8. Auflage , Seiten 69, 1969 und 4740

## Beschreibung

Die vorliegende Erfindung betrifft ein Verfahren zur Herstellung von Nitrobenzol durch isotherme Nitrierung von Benzol mit einem Salpetersäure-Schwefelsäure-Gemisch. Abtrennung des gebildeten Nitrobenzols, Eindampfung der Schwefelsäure und Rückführung der aufkonzentrierten Schwefelsäure zur Benzolnitrierung.

Die Herstellung von Nitrobenzol geschieht durch Nitrierung von Benzol mittels sogenannter Nitriersäure, einem Gemisch aus Salpetersäure und Schwefelsäure

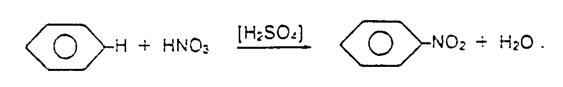

Die Schwefelsäure bindet das in diesem Prozess gebildete Reaktionswasser. Durch das fortwährend gebildete Wasser und den Verbrauch der Salpetersäure bei der obigen Reaktion entsteht aus der Nitriersäure eine relativ verdünnte Schwefelsäure. Zur Aufrechterhaltung einer nötigen Arbeitskonzentration muss daher verdünnte Säure, sogenannte Absäure, aus dem System abgeführt werden und durch konzentrierte Säure ersetzt werden. Dies stellt einen wesentlichen Kostenfaktor im Herstellungsprozess dar. Darüberhinaus bereitet der Verbleib der Absäure, die durch organische Verbindungen und Stickstoffoxide verunreinigt ist, erhebliche Probleme. Die Verwendung der Absäure in der Düngemittelindustrie setzt entsprechende Massnahmen voraus, um die an die Reinheit der Dünnsäure gestellten Anforderungen zu erfüllen (US-Patentschrift 4 257 986).

Eine Möglichkeit, die Menge der anfallenden Absäure zu verringern, besteht darin, dass anstelle von konzentrierter Schwefelsäure Oleum als Frischsäure in den Prozess eingebracht wird, wobei Oleum aber naturgemäss teurer ist als Schwefelsäure.

Alternativ wurde das Recycling der Säure angestrebt. Die anfallende Absäure wird in einer Hochkonzentrierung auf einen H₂S0₄-Gehalt von 95 bis 97% bei Normaldruck eingedampft. Dabei werden die organischen Verbindungen weitgehend verdampft oder oxidativ zerstört, so dass eine relativ reine Säure in den Prozess zurückgeführt werden kann (EP-A-16 987). Dieses Verfahren ist aufgrund der hohen Temperaturen und der anfallenden Investitionskosten für die Hochkonzentrieranlage sehr kostenintensiv.

Ein weiterer Weg zur Verbesserung der Wirtschaftlichkeit des Verfahrens zur Herstellung von Nitrobenzol ist eine adiabatische Prozessführung bei der Nitrierung.

Die entstehende Reaktionswärme wird also nicht durch Kühlung während des Prozesses abgeführt, sondern anschliessend zur Verdampfung des Reaktionswassers genutzt, so dass man direkt eine rezirkulierbare Schwefelsäure erhält. Allen hierzu vorgeschlagenen Prozessen (US-PS 3 928 475; US-PS 3 981 935; EP-PS 39 556; US-PS 4 021 498; US-PS 4 091 042) ist gemeinsam, dass sie neue Anlagen zur Durchführung der Prozesse erfordern, bei denen durch die hohen Prozesstemperaturen (bis 145 _{°} C) der Einsatz spezieller korrosionsbeständiger Werkstoffe und wesentlich erhöhte Sicherheitsmassnahmen erforderlich werden. Dadurch werden die potentiellen Vorteile dieser Prozesse kompensiert.

Das Ziel der vorliegenden Erfindung ist es, die Herstellung von Nitrobenzol nach den üblichen kontinuierlichen oder diskontinuierlichen isothermen Verfahren so zu verbessern, dass gegenüber dem Stand der Technik wesentliche wirschaftliche und ökologische Vorteile erreicht werden.

Überraschenderweise wurde nun gefunden, dass man auch bei einem vollständigen Recycling der Schwefelsäure über längere Zeiträume auf eine zusätzliche Reinigung verzichten kann, ohne dass der Prozess der Nitrobenzolherstellung negativ beeinflusst wird, wenn die anfallende Absäure einer Mittelkonzentrierung in Horizontalverdampfern unter Vakuum bei Temperaturen bis maximal 195°C unterworfen wird.

Gegenstand der vorliegenden Erfindung ist somit ein Verfahren zur Herstellung von Nitrobenzol durch isotherme Nitrierung von Benzol mit einem Salpetersäure-Schwefelsäure-Gemisch. Abtrennung des gebildeten Nitrobenzols. Eindampfung der Schwefelsäure und Rückführung der aufkonzentrierten Schwefelsäure zur Benzolnitrierung, wobei die Schwefelsäure durch Mittelkonzentrierung in einem oder mehreren hintereinandergeschalteten Horizontalverdampfern unter Vakuum bei Temperaturen zwischen 130 und 195 _{°} C auf eine Konzentration von 75 bis 92%, vorzugsweise 80 bis 90% ohne zusätzliche Reinigung eingedampft und rückgeführt wird.

Durch Anwendung des erfindungsgemässen Verfahrens ist es somit möglich, die Eindampfung unter Vakuum mit Dampf durchzuführen bei Temperaturen, die den Einsatz der äusserst korrosionsbeständigen Werkstoffe Tantal, Glas, emaillierter Stahl und Teflon gestatten. Als Verdampfer werden erfindungsgemäß Horizontalverdampfer eingesetzt.

Neben der einfachen Bau- und Betriebsweise bieten letztere den Vorteil, dass infolge der Mehrstufigkeit die Wasserverdampfung zum überwiegenden Teil bei so geringen Schwefelsäurekonzentrationen erfolgt, dass selbst eine Eindampfung auf einen H₂S0₄-Gehalt von 92% ohne Rektifikation der Brüden möglich ist. Die Schwefelsäureverluste liegen dabei maximal bei 1 %. Gleichzeitig ergibt sich infolge des Blasensiedens an den Heizrohren, die im allgemeinen aus Tantal sind, eine sehr hohe spezifische Verdampfungsleistung, was bei den hohen Kosten der Tantalwärmetauscher ein eminenter Vorteil ist.

Besonders bevorzugt ist somit die Ausführungsform des erfindungsgemässen Verfahrens, in der die Eindampfung in einem oder mehreren hintereinandergeschalteten Horizontalverdampfern erfolgt.

Die Effizienz des Verfahrens kann wesentlich verbessert werden, indem durch den Einbau von Trennwänden in die Horizontalverdampfer eine mindestens 3-stufige Eindampfung erfolgt, besonders bevorzugt ist eine mindestens 5-stufige Eindampfung. Die Brüden aus der Eindampfung werden unmittelbar ohne Rektifikation durch direkte oder indirekte Kühlung kondensiert.

Auf eine aufwendige Reinigung der Kreislaufsäure kann verzichtet werden. Besonders vorteilhaft ist es, wenn die verdünnte Schwefelsäure durch die aufkonzentrierte Säure aufgeheizt wird. Dies kann geschehen, indem die kalte Absäure im Gegenstrom zur heissen eingedampften Schwefelsäure durch Glas- oder Teflonwärmetauscher geschicht und somit auf ca. 100°C aufgeheizt wird.

Das Verfahren wird vorteilhaft so durchgeführt, dass aus der aufgeheizten verdünnten Schwefelsäure durch Evakuieren Benzol, Nitrobenzol und Wasser verdampft werden, bevor die verdünnte Schwefelsäure in den Horizontalverdampfer eingebracht wird. Auf diese Weise kann eine Beeinträchtigung der Säureeindampfung im Verdampfer ausgeschlossen werden. Eine besondere Ausführungsform des erfindungsgemässen Verfahrens besteht darin, dass die kondensierten Brüden mit Dampf gestrippt werden und Benzol und Nitrobenzolnitrierung zurückgeführt werden. Alternativ können die Nitrokörper mit dem zur Nitrobenzolherstellung eingesetzte Benzol extrahiert und das benzolhaltige Brüdenkondensat einer Abwasserbehandlung zugeführt werden.

Das erfindungsgemässe Verfahren bietet weitere Vorteile durch seine Einfachheit. Überraschend wurde gefunden, dass weder eine Abtrennung oder Zerstörung der organischen Verbindungen für den ungestörten Ablauf der Nitrobenzolherstellung bei vollständiger Schwefelsäurezirkulierung erforderlich ist, noch die weitgehende Entfernung der Metallsulfate, die mit der Salpetersäure und durch Korrosion in die Schwefelsäure gelangen. Erfindungsgemäss werden die aus der konzentrierten Säure kristallisierenden Metallsulfate durch periodisches Spülen des Säure-Säure-Wärmetauschers aus dem System entfernt. Eine Beeinträchtigung der Wärmeaustauschleistung und des Säuredurchflusses können somit ausgeschaltet werden.

Das Arbeiten mit einer Kreislaufsäure, die relativ viel gelöste Schwermetallsulfate enthält, bietet den Vorteil, dass der NOₓ₋Pegel gegenüber frischer Säure abgesenkt wird. Ausserdem wird die Korrosionsrate in den Reaktionsapparaten durch die relative Sättigung der Säure mit Metallsulfaten gesenkt.

Die Wirtschaftlichkeit des Verfahrens zur Herstellung von Nitrobenzol lässt sich erfindungsgemäss dadurch wesentlich verbessern, dass die Konzentration der im Salpetersäure-Schwefelsäure-Gemisch eingesetzten Salpetersäure im Bereich von 60 bis 70% liegt. Eine Salpetersäure dieser Konzentration ist wesentlich preiswerter als die üblicherweise eingesetzte 99%ige Salpetersäure. Auf diese Weise kann die kostenintensive extraktive Salpetersäuredestillation ersetzt werden durch die erfindungsgemässe Wasserverdampfung aus Schwefelsäure unter optimalen Bedingungen. Der Einsatz von 60 bis 70%iger Salpetersäure bietet ausserdem den Vorteil, dass diese sowohl weniger NO_{X} als auch weniger Nitrate, insbesondere Aluminiumnitrat, enthält.

Die wesentlichen Vorteile des erfindungsgemässen Verfahrens sind darin zu sehen, dass die seit Jahrzehnten bewährten vorhandenen Anlagen zur isothermen kontinuierlichen oder diskontinuierlichen Herstellung von Nitrobenzol weiterhin benutzt werden können. Es ist weiterhin eine völlige Rückführung der für den Nitrierprozess benötigten Schwefelsäure möglich.

Die Verdampfung des Reaktionswassers erfolgt unter Vakuum bei maximal 195_{°}C, so dass praktisch keine Säureverluste eintreten und relativ wenig Sekundärenergie in Form von Dampf benötigt wird.

Durch den Einsatz von 60 bis 70%iger Salpetersäure anstelle von 98 bis 99%iger Salpetersäure werden die Menge der bei der Schwefelsäureeindampfung abgeschiedenen Metallsalze und die Menge des freiwerdenden NO_{X} verringert, sowie die Verdampfung des Verdünnungswassers auf einem niedrigen und damit energetisch günstigen Konzentrationsniveau durchgeführt.

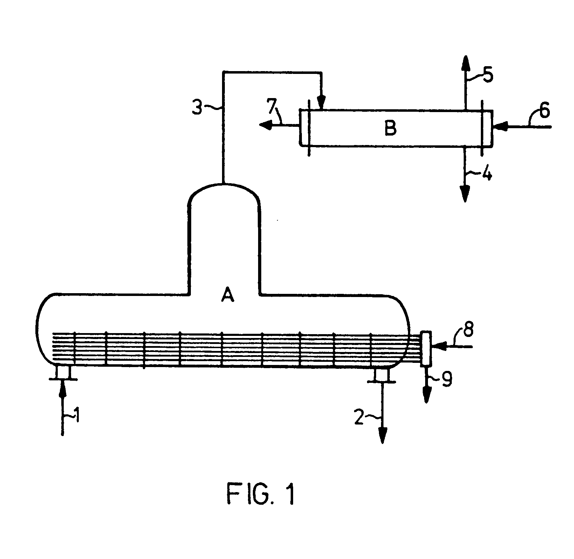

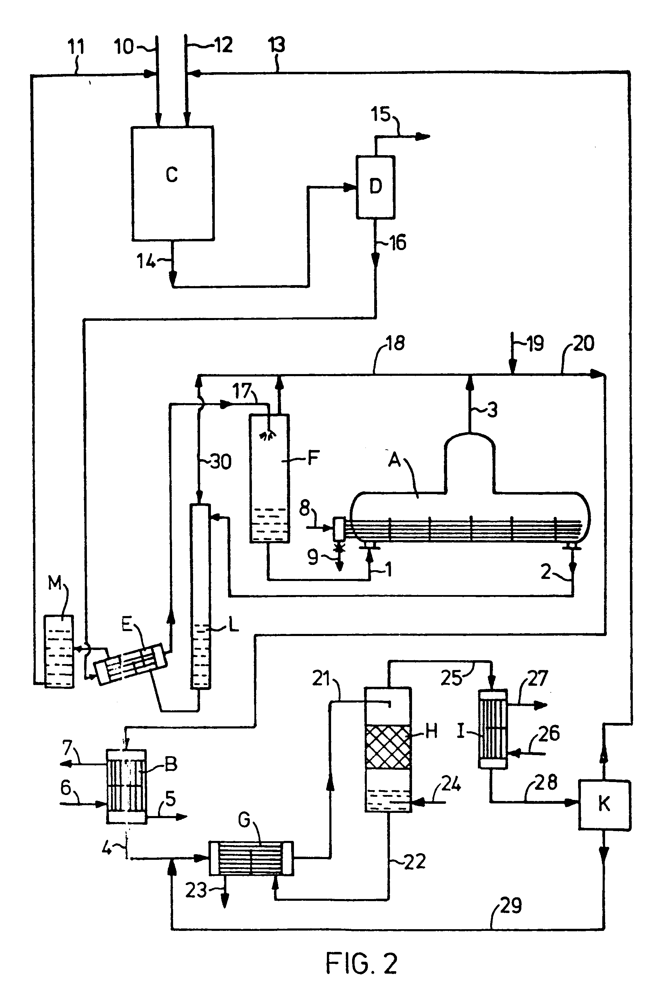

Das erfindungsgemässe Verfahren soll anhand der Abbildungen näher erläutert werden. Fig. 1 zeigt den Horizontalverdampfer zur Eindampfung der Schwefelsäure unter Vakuum. In Fig. 2 ist beispielhaft der Verbund von Schwefelsäureeindampfung und Nitrobenzolherstellung dargestellt.

In Fig. 1 besteht der Horizontalverdampfer (A) aus einem zylindrischen liegenden Gefäss aus Glas oder emailliertem Stahl mit einem aufgesetzten Brüdendom, in das ein Bündel aus Tantalrohren eingesteckt ist. Die Dampfzuleitung (8) und die Kondensatableitung (9) erfolgen auf der gleichen Seite. Alternativ kann auch ein Rohrbündel eingebaut werden, bei dem auf der einen Seite Dampf zugeführt und auf der anderen Seite das Kondensat abgeführt wird. Die verdünnte 65 bis 75%ige Schwefelsäure (1) wird an einem Ende vorzugsweise von unten in den Verdampfer eingespeist und fliesst axial durch den Verdampfer. Die Ausspeisung der 75 bis 92%igen Säure (2) wird so geregelt, dass die Tantalrohre immer mit Säure bedeckt sind. Zwischen den eingebauten Trennwänden aus Teflon erfolgt eine starke Vermischung der Säure infolge der Blasenbildung beim Sieden. Von einem Verdampferabschnitt zum nächsten erfolgt aber jeweils ein sprunghafter Konzentrationsanstieg. Damit ist der Vorteil verbunden, dass nur am auslaufseitigen Ende des Verdampfers merkliche Mengen Schwefelsäuredampf in den Brüden enthalten sind, und das auch nur dann, wenn die Konzentration der ausgespeisten Säure (2) über 90% liegt. Die Brüden (3) werden aus dem Dom in einen Kondensator (B) geleitet. Der Kondensator wird mit Kühlwasser (6) beaufschlagt, das als unbelastetes Abwasser (7) abfliesst. Das Brüdenkondensat (4) wird einer weiteren Behandlung zugeführt. Die nichtkondensierbaren Gase (5) werden mit einer Vakuumpumpe abgezogen. Die Brüdenkondensation kann alternativ durch direkten Kontakt der Brüden mit Brüdenkondensat erfolgen, das über einen Kühler im Kreislauf gefördert wird.

Den vorteilhaften Verbund von Nitrobenzolherstellung und Schwefelsäureeindampfung zeigt Fig. 2. In die Benzolnitrierung (C) werden Salpetersäure (10), eingedampfte Kreislaufschwefelsäure (11), Benzol (12) und das aus der gebrauchten Schwefelsäure (16) zurückgewonnene Benzol-Nitrobenzol-Gemisch (13) eingespeist. Bei Einsatz von 99%iger Salpetersäure (10) ist im allgemeinen eine Konzentration von 80 bis 85% für die Schwefelsäure (1) ausreichend. Wird 60 bis 70%ige Salpetersäure (10) verwendet, empfiehlt sich die Eindampfung der Schwefelsäure auf 90 bis 93% H₂S0₄, um die Benzolnitrierung (C) nicht durch zu grosse Flüssigkeitsmengen zu überlasten. Die bei der Eindampfung anzustrebende Säurekonzentration richtet sich demzufolge nach der hydrodynamischen Belastbarkeit der Benzolnitrieranlage. Das aus der Nitrierung ausgespeiste Nitrobenzol-Schwefelsäure-Gemisch (14) wird in den üblichen Trennapparaturen (D) in Roh-Nitrobenzol (15) und verdünnte Schwefelsäure (16) getrennt. Eine weitgehende Austreibung des Benzols aus der Schwefelsäure (16) ist empfehlenswert, aber nicht notwendig. Desgleichen ist eine Zerstörung der anorganischen Nitroverbindungen durch Umsetzung mit Schwefeldioxid, Harnstoff, Ammoniumsulfat, Amidosulfonsäure oder Strippen mit Dampf möglich, aber im Sinne der Erfindung nicht erforderlich. Die Schwefelsäure (16) wird mit 30 bis 60 _{°} C in den Wärmetauscher (E) eingeleitet, den sie mit 90 bis 120 _{°} C verlässt (17). In dem Entspannungsverdampfer (F) verdampfen bei Unterdruck nahezu alles Benzol sowie ein Teil des Wassers und Nitrobenzols (18). Die weitgehend benzolfreie Schwefelsäure (1) wird in den Horizontalverdampfer (A) eingeleitet, in dem sie bei 10 bis 200 mbar und 130 bis 195 ° C auf eine H₂S0₄-Konzentration von 75 bis 92% eingedampft wird. Die heisse konzentrierte Säure (2) wird im Wärmetauscher (E) zur Aufheizung der verdünnten Säure (16) benutzt. Die konzentrierte Säure (2) fliesst in ein Zulaufrohr (L), das über eine Gaspendelleitung (30) mit dem Unterdrucksystem verbunden ist. Durch den siphonartigen Zulauf wird ein bestimmtes Säureniveau im Horizontalverdampfer gewährleistet. Aus dem Zulaufrohr (L) tritt die Säure (2) in den Wärmetauscher (E) ein. Die auf 50 bis 70 _{°} C gekühlte austretende Säure (11) kann in einem weiteren Wärmetauscher mit Wasser auf 30 bis 50 _{°} C abgekühlt werden, bevor sie in die Vorlage (M) gelangt, aus der sie nach Bedarf in die Benzolnitrierung (C) eingespeist wird. Der Heizdampf (A) des Horizontalverdampfers soll höchstens eine Temperatur von 220 _{°} C haben, um eine Gefährdung des Tantals durch Korrosion auszuschliessen. Das Dampfkondensat (9) kann vorteilhaft zur Dampferzeugung eingesetzt werden. Ausserdem kann durch Entspannungsverdampfung Dampf für das Strippen des Brüdenkondensats gewonnen werden. Die Brüden (3) aus dem Horizontalverdampfer (A) werden zusammen mit den Brüden (18) aus dem Entspannungsverdampfer (F) dem Kondensator (B) zugeleitet. Dabei erfolgt vorzugsweise durch Einspritzen (19) von Wasser oder Brüdenkondensat (4) eine Abkühlung der überhitzten Brüden auf Sattdampftemperatur (20). Im Kondensator (B) erfolgt die Brüdenkondensation durch indirekte Kühlung mit einer Kühlflüssigkeit (6), vorzugsweise Wasser. Die Temperatur der aus dem Kondensator (B) ausfliessenden Kühlflüssigkeit (7) bestimmt den Unterdruck im Verdampfungssystem. Die nichtkondensierbaren Gase (5) werden mit einer Vakuumpumpe abgezogen und einer Abgasreinigung zugeleitet. Das Brüdenkondensat (4) wird in einem Wärmetauscher (G) durch das gestrippte Abwasser (22) aufgeheizt (21) und in eine Strippkolonne (H) eingespeist, in der es mit Dampf (24) weitgehend benzol-und nitrobenzolfrei gestrippt wird. Aus der Kolonne (H) fliesst es, nachdem es im Wärmetauscher (G) durch kaltes Brüdenkondensat (4) abgekühlt wurde (23) zur Abwasserbehandlung. Der Dampf (25) aus der Strippkolonne (H) wird im Wärmetauscher (I) mittels Kühlwasser (26, 27) kondensiert. Das Kondensat (28) wird in einem Scheidegefäss (K) in eine wässrige Phase (29), die wieder zur Strippung zurückgeführt wird, und in eine organische Phase (13) getrennt, die im wesentlichen aus Benzol und Nitrobenzol besteht und zur Nitrierung zurückgeführt wird.

Anstelle der umweltbewussten, aber aufwendigen Strippung des Brüdenkondensats ist auch eine Benzolwäsche zur Entfernung des Nitrobenzols und eine biologische Reinigung des benzolhaltigen Abwassers möglich.

Die beim Abkühlen aus der konzentrierten Säure (2) ausgeschiedenen Metallsulfate lagern sich im Wärmetauscher (E) ab. Dadurch erhöht sich der Strömungswiderstand und das Säureniveau im Zulaufrohr (L) steigt. Erfindungsgemäss wird der Wärmetauscher (E) entleert, wenn der Flüssigkeitsspiegel im Zulaufrohr (L) etwa die Höhe des Säurezulaufs in das Rohr erreicht hat. Der Wärmetauscher wird durch Spülen mit Wasser oder verdünnter Säure gereinigt und die Anlage wieder in Betrieb genommen.

Wenn grosse Wassermengen aus der verdünnten Schwefelsäure zu verdampfen sind oder wenn eine Eindampfung bis auf 92% H₂S0₄ erforderlich ist, bietet die Hintereinanderschaltung mehrerer Horizontalverdampfer Vorteile. Der Druck, bei dem die Verdampfung in den einzelnen Verdampfern erfolgt, soll um so geringer sein, je höher die Konzentration der ausgespeisten Schwefelsäure ist.

Die Vorteile des erfindungsgemässen Verfahrens (Beispiele 2 und 3) sollen durch den Vergleich mit einem herkömmlichen Verfahren (Beispiel 1) verdeutlicht werden, ohne dass durch die angeführten Beispiele eine Einschränkung des Umfanges der Erfindung erfolgt.

### Beispiel 1 (Vergleichsbeispiel)

Absäure aus der Benzolnitrierung mit 70% H₂S0₄, 0,05% Benzol, 0,03% Nitrobenzol und 0,08% NO_{X} wurde in einem Pauling-Plinke-Kessel auf 96% H₂S0₄ eingedampft und zur Benzolnitrierung zurückgeführt. Bei der Benzolnitrierung wurden 645 kg/h aufkonzentrierte 96%ige Schwefelsäure, 775 kg/h 99%ige HN0₃ und 1000 kg/h Benzol eingesetzt, entsprechend ca. 5% Benzolüberschuss. Die anfallende 70%ige Absäure (872 kg/h) wurde über einen Wärmetauscher, der mit den Verdampferbrüden beheizt wurde, in den Dephlegmator eines Pauling-Plinke-Verdampfers eingespeist. Der Kessel des Verdampfers wurde mit einem Erdgasbrenner befeuert. Der Gasverbrauch lag bei 64 m³ₙ/h. Die mit 330 ° C aus dem Kessel ausfliessende 96%ige Schwefelsäure wurde in einem Rührkühler auf 50 ° C abgekühlt und in einen Stapeltank geleitet. Im Stapeltank setzen sich Metallsulfate ab. Die zur Benzolnitrierung zurückgeführte Säure war farblos.

Die Brüden aus dem Kopf der Dephlegmatorkolonne enthielten neben Wasserdampf 0,44 kg/h Benzol, 0,26 kg/h Nitrobenzol, 0,35 kg/h NO_{X} (als N0₂ gerechnet) und Spuren S0₂. Nach der Brüdenkondensation wurde die organische Phase durch Strippen (s. Beispiel 2) abgetrennt und zur Benzolnitrierung zurückgeführt (0,44 kg/h Benzol und 0,25 kg/h Nitrobenzol). Die Schwefelsäureverluste betrugen 3%.

Der Energieverbrauch lag bei 8400 kJ/kg H₂0-Verdampfung.

### Beispiel 2

Das Beispiel betrifft das erfindungsgemässe Verfahren unter Bezug auf Abb. 2.

In die Benzolnitrierung (C) wurden 4470 kg/h aufkonzentrierte Schwefelsäure (11) mit 82,5% H₂ S0₄ und 2575 kg/h 99%ige HN0₃ (10) eingespeist. Gleichzeitig wurden 3313 kg/h Benzol (12) und 80 kg/h Benzol/Nitrobenzol-Gemisch (13) mit ca. 33% Benzolanteil eingespeist, entsprechend ca. 5% Benzolüberschuss. Das ausgespeiste Gemisch (14) wurde in Rohnitrobenzol (15) und Absäure (16) getrennt.

Die Absäure 5220 kg/h mit 70% H₂S0₄, wurde in mehreren hintereinandergeschalteten Rohrenwärmetauschern (E) aus Glas durch die aufkonzentrierte Schwefelsäure (2) auf 107 ° C vorgeheizt. Diese vorgeheizte Säure (17) wurde in den Flash-Verdampfer (F) eingespeist, in dem die Hauptmenge des Benzol und Nitrobenzols und so viel Wasser verdampften, dass noch 5170 kg/h Säure (1) mit 100_{°}C in den Horizontalverdampfer (a) eingespeist wurden. Das Tantal-Rohrbündel des Horizontalverdampfers wurde mit Sattdampf von 180° C (B) beheizt. Der Dampfverbrauch lag bei 1300 kg/h, entsprechend 4940 kJ/kg H₂0-Verdampfung, wenn das Dampfkondensat (9) nicht genutzt wurde. Bei Ausnutzung des Dampfkonzentrats zur Dampferzeugung ergab sich ein spezifischer Energiebedarf von 3475 kJ/kg H₂0-Verdampfung.

Die Verdampfung des Wassers erfolgte 5-stufig bei einem Druck von 133 mbar, wobei die Temperatur bis auf 160_{°}C in der 5. Stufe anstieg. Die aufkonzentrierte Säure (2) floss über das Zulaufrohr (L) in die Wärmetauscher (E) ab, in denen sie auf ca. 60° C durch die Absäure (16) abgekühlt wurde. In einem wassergekühlten Wärmetauscher (nicht dargestellt) erfolgte eine weitere Abkühlung auf 40 °C, bevor die Säure (11) aus Vorlage (M) zur Benzolnitrierung (C) zurückgefördert wurde.

In die überhitzten Brüden (3) aus dem Horizontalverdampfer (A) wurden 200 I/h Brüdenkondensat (4) eingedüst, wodurch die Temperatur der Brüden auf 51 _{°} C gesenkt wurde. Die Kondensation der Brüden aus dem Flash-Verdampfer (18) und dem Horizontalverdampfer (3) erfolgte in einem wassergekühlten Röhrenwärmetauscher (B). Es fielen 790 kg/h Brüdenkondensat mit 40° C an. Die nichtkondensierbaren Gase (Leckage-Luft, 0,6 kg/h Benzol, 0,03 kg/h Nitrobenzol, 0,1 kg/h NOₓ) wurden mit einer Wasserringpumpe abgesaugt und einer Abgas-Verbrennung zugeführt.

Das Brüdenkondensat enthielt 2,2 kg H₂S0₄/t, 2,7 kg Benzol/t und 2 kg Nitrobenzol/t Kondensat. Es wurde im Wärmetauscher (G) auf ca. 90° C aufgeheizt und in den Stripper (H) eingespeist. In diesem erfolgte die Strippung durch Einleiten von 50 kg/h 5 bar Dampf (24) in den Sumpf. Das gestrippte Brüdenkondensat 22) wurde im Wärmetauscher (G) auf 45° C gekühlt und als Abwasser abgeführt. Der benzol- und nitrobenzolhaltige Dampf aus dem Stripper (25) wurde im Kondensator (J) kondensiert und in einer Scheideflasche (K) in eine organische und eine wässrige Phase getrennt. Die wässrige Phase (29) wurde mit dem Brüdenkondensat (4) vereinigt und dem Stripper zugeleitet. Die organische Phase (ca. 2,7 kg/h Benzol und 2 kg/h Nitrobenzol) wurde in die Nitrierung (C) zurückgeführt.

Nachdem die Anlage 5-6 Monate im Verbund betrieben worden war, hatte sich ein stationärer Zustand eingestellt. Die Säure war durch Eisen-, Chrom-, Nickel-sulfat und andere Sulfate schwarzgrün gefärbt. Im Mantelraum des Wärmetauschers (E) schieden sich ständig Aluminiumsulfat und andere Metallsulfate ab. Sie mussten durch regelmässiges Spülen mit Wasser alle 3-4 Tage entfernt werden, wofür die Eindampfung 2-3 h lang unterbrochen wurde. Während des Verbundbetriebes war der durchschnittliche NOₓ₋Gehalt in der aufkonzentrierten Säure (11) von anfänglich 1000 ppm auf 400 ppm abgesunken. Die H₂S0₄-Verluste während der Eindampfung lagen mit 0,05% (bez. auf H₂S0₄) sehr niedrig. Bei der Benzolnitrierung wurden keine Nachteile durch die Schwefelsäurerecyclierung festgestellt.

### Beispiel 3

Benzolnitrierung und Schwefelsäureeindampfung erfolgten analog Beispiel 2 mit folgenden Unterschieden: Als Salpetersäure (10) wurde 67%ige HN0₃ eingesetzt. Die Schwefelsäure wurde von 70 auf 92% H₂S0₄ eingedampft. Für die Eindampfung waren zwei Verdampfer (A) parallel geschaltet, die mit Sattdampf (8) von 195_{°}C beheizt wurden. Der Druck in den Verdampfern lag bei 40 mbar, die Temperatur der abfliessenden Säure (2) bei 182° C.

Es wurden 3500 kg/h Absäure (16) mit 70% H₂S0₄, 0,05% Benzol, 0,03% Nitrobenzol und 0,01% N0₂ nach Vorwärmung auf 110°C in den Flash-Verdampfer (F) eingespeist. Aus den Horizontalverdampfern (A) wurden insgesamt 2640 kg/h 92%ige Schwefelsäure (2) mit 182° C ausgespeist. Die Brüden wurden analog Beispiel 2 aufgearbeitet. Nachdem sich ein stationärer Betriebszustand eingestellt hatte, musste der Wärmetauscher (E) mantelseitig alle 15-20 Tage gespült werden. Der NOₓ₋Gehalt der aufkonzentrierten Säure (11) lag bei 0,006%. Das Abgas enthielt ca. 1 g NO_{X}/h.

## Patentansprüche

1. Verfahren zur Herstellung von Nitrobenzol durch isotherme Nitrierung von Benzol mit einem Salpetersäure-Schwefelsäure-Gemisch, Abtrennung des gebildeten Nitrobenzols, Eindampfung der Schwefelsäure und Rückführung der aufkonzentrierten Schwefelsäure zur Benzolnitrierung, dadurch gekennzeichnet, dass die Schwefelsäure durch Mittelkonzentrierung in einem oder mehreren hintereinandergeschalteten Horizontalverdampfern unter Vakuum bei Temperaturen zwischen 130 und 195_{°}C auf eine Konzentration von 75 bis 92%, vorzugsweise 80 bis 90%, ohne zusätzliche Reinigung eingedampft und rückgeführt wird.

2. Verfahren gemäss Anspruch 1, dadurch gekennzeichnet, dass durch den Einbau von Trennwänden in die Horizontalverdampfer eine mindestens 3-stufige Eindampfung erfolgt.

3. Verfahren gemäss Anspruch 2, dadurch gekennzeichnet, dass eine mindestens 5-stufige Eindampfung erfolgt.

4. Verfahren gemäss einem der Ansprüche 1 bis 3, dadurch gekennzeichnet, dass die Brüden aus der Eindampfung ohne Rektifikation unmittelbar durch direkte Kühlung kondensiert werden.

5. Verfahren gemäss einem der Ansprüche 1 bis 4, dadurch gekennzeichnet, dass die verdünnte Schwefelsäure durch die aufkonzentrierte Säure aufgeheizt wird.

6. Verfahren gemäss einem der Ansprüche 1 bis 5, dadurch gekennzeichnet, dass die Konzentration der im Salpetersäure-Schwefelsäure-Gemisch eingesetzten Salpetersäure im Bereich von 60 bis 70% liegt.

## Claims

1. Process for the production of nitrobenzene by isothermal nitration of benzene with a mixture of nitric acid and sulphuric acid, separation of the nitrobenzene formed, concentration of the sulphuric acid by evaporation and return of the concentrated sulphuric acid to the plant for nitration of benzene, characterised in that the sulphuric acid is concentrated by evaporation to a concentration of from 75 to 92%, preferably from 80 to 90%, under vacuum at temperatures of between 130 and 195 °C by medium concentration in one or more horizontal evaporators connected in series without additional purification and is recycled.

2. Process according to claim 1, characterised in that concentration by evaporation is carried out in at least three stages by the incorporation of partitions into the horizontal evaporators.

3. Process according to claim 2, characterised in that concentration by evaporation takes place in at least five stages.

4. Process according to one of claims 1 to 3, characterised in that the vapours from the concentration by evaporation are immediately condensed by direct cooling without rectification.

5. Process according to one of claims 1 to 4, characterised in that the diluted sulphuric acid is heated by the acid which has been concentrated.

6. Process according to one of claims 1 to 5, characterised in that the concentration of the nitric acid used in the mixture of nitric acid and sulphuric acid is in the range of 60 to 70%.

## Revendications

1. Procédé de préparation de nitrobenzène par nitration isotherme de benzène avec un mélange d'acide nitrique/acide sulfurique, séparation du nitrobenzène formé, évaporation de l'acide sulfurique et récupération de l'acide sulfurique concentré pour la nitration du benzène, caractérisé en ce qu'on évapore l'acide sulfurique par concentration moyenne dans un ou plusieurs évaporateurs horizontaux montés l'un derrière l'autre, sous vide, à des températures comprises entre 130 et 195_{°}C, jusqu'à une concentration de 75 à 92%, de préférence, de 80 à 90%, sans purification additionnelle et puis on le récupère.

2. Procédé selon la revendication 1, caractérisé en ce qu'une évaporation au moins en trois étapes a lieu en installant des cloisons dans des évaporateurs horizontaux.

3. Procédé selon la revendication 2, caractérisé en ce qu'on effectue une évaporation au moins en cinq étapes.

4. Procédé selon l'une quelconque des revendications 1 à 3, caractérisé en ce que les vapeurs chaudes provenant de l'évaporation sont condensées directement par refroidissement direct sans rectification.

5. Procédé selon l'une quelconque des revendications 1 à 4, caractérisé en ce que l'acide sulfurique dilué est chauffé par l'acide concentré.

6. Procédé selon l'une quelconque des revendications 1 à 5, caractérisé en ce que la concentration de l'acide nitrique introduit dans le mélange d'acide nitrique/acide acide sulfurique se situe dans l'intervalle allant de 60 à 70 %.
